# EUROPEAN PATENT SPECIFICATION

(11) **EP 3 156 924 B1**
(45) Date of publication and mention of the grant of the patent: **29.07.2020**
(21) Application number: 16162083.6
(22) Date of filing: 23.03.2016
(51) Int. Cl.: G16H 20/30

(54) **METHOD AND DEVICE FOR DETERMINING VALUE OF CONSUMED ENERGY**
VERFAHREN UND VORRICHTUNG ZUR BESTIMMUNG DES WERTS VON VERBRAUCHTER ENERGIE
PROCÉDÉ ET DISPOSITIF DE DÉTERMINATION DE VALEUR DE CONSOMMATION D'ÉNERGIE

(30) Priority: 12.10.2015 CN 201510660649
(43) Date of publication of application: 19.04.2017
(73) Proprietor: Xiaomi Inc., Beijing 100085 (CN)
(72) Inventor: WU, Ke, 100085 BEIJING (CN); LIU, Xinyu, 100085 BEIJING (CN); LIU, Huayijun, 100085 BEIJING (CN)
(74) Representative: Cabinet Beau de Loménie

(56) References cited:
- WO-A1-2014/098628

## Description

### TECHNICAL FIELD

The present invention relates to the field of computer technologies, and more particularly, to a method and device for determining a value of consumed energy.

### BACKGROUND

People have begun to pay attention to health problems with the continuous improvement of living standards, and most people are keen on keeping fit by means of exercises. Generally, people will choose to calculate a value of energy consumed in an exercise to determine an amount of the exercise.

When determining an amount of an exercise, people will generally record the name and a corresponding duration of a single exercise and then inquire a value of energy that can be consumed in the exercise within a unit of time, e.g. a value of energy consumed by sit-ups performed within 1 minute is 10 calories. Thus, a value of energy consumed by an exercise may be calculated according to an exercise duration, and an amount of a single exercise may be determined accordingly.

The inventors find that at least the following problems exist during implementation of the present invention.

In the calculation method above, a value of energy consumed by an exercise is only related to a duration of the exercise without considering that there may be a significant difference in energy consumption due to different exercise amplitudes and exercise frequencies within the same period of time. Thus, the accuracy in calculating a value of energy consumed by an exercise will be greatly reduced by calculating the value of energy consumed by the exercise according to an exercise duration.

Document WO 2014/098628A1 discloses a method and a system of measuring an activity of a person on a flexible mat of a trampoline, the method comprising: determining a mat deformation signal based at least on measurements from a sensor arrangement comprising at least one sensor and defining a coordinate space for the mat; the or each sensor being configured to measure a value corresponding to a deformation of the mat as the person/object moves on the mat; wherein, the value measured by die or each sensor corresponds to a proximity of the person/object to the sensor; determining a bounce coordinate in the coordinate spate based at least on the mat deformation signal; and determining a bounce location of the person/object on the mat based at least on the bounce coordinate in the coordinate space.

### SUMMARY

Accordingly, the present invention provides a method and device for determining a value of consumed energy, in accordance with claims which follow.

According to a first aspect of the embodiments of the present invention, a method for determining a value of consumed energy is provided in accordance with claim 1.

Thus, in the embodiments of the present invention, deformation information of the surface of an elastic mat is acquired; a value of energy consumed by a user when corresponding deformation is generated is determined according to the deformation information of the surface of the elastic mat; and the value of energy is recorded. Thus, the user will press the surface of the elastic mat in different degrees accordingly when doing exercises on the elastic mat, and the elastic mat can specifically determine, according to deformation information of the surface of the elastic mat, a value of energy consumed by the user when corresponding deformation is generated, so as to improve the accuracy in calculating a value of energy consumed via an exercise.

In this way, the value of the energy consumed by the user may be determined according to the deformation degree and the deformation duration of each detection point of the surface of the elastic mat, thus improving the accuracy in calculating a value of energy consumed via an exercise.

According to an exemplary embodiment, the method may further include that:
based on deformation degrees, deformation durations and preset adjustment coefficients, a calculation formula of work on detection points of the elastic mat is constructed;
for each detection point of the elastic mat, values of the adjustment coefficients in the calculation formula are determined according to the deformation degree and a deformation duration of the detection point and a value of work on the detection point during actual detection, so as to obtain a calculation formula having determined coefficients and corresponding to each detection point;
the step that a value of work by the user on the elastic mat at each detection point of the elastic mat is determined according to the deformation degree and the deformation duration of each preset detection point of the surface of the elastic mat may include that:
   a value of work by the user on the elastic mat at each detection point of the elastic mat is determined according to the deformation degree and the deformation duration of each preset detection point of the surface of the elastic mat and a calculation formula having determined coefficients and corresponding to each detection point.

In this way, a calculation formula for work on the elastic mat is acquired according to actual detection, which can calculate a value of the work of the user on the elastic mat more accurately.

According to an exemplary embodiment, the step that values of the adjustment coefficients in the calculation formula are determined for each detection point of the elastic mat according to the deformation degree and the deformation duration of the detection point and a value of work on the detection point during actual detection, so as to obtain a calculation formula having determined coefficients and corresponding to each detection point may include that:
values of the adjustment coefficients in the calculation formula are determined for each detection point of the elastic mat according to the deformation degree and the deformation duration of the detection point and a value of work on the detection point during actual detection under each exercise type, so as to obtain a relationship among the exercise type, the detection point and a calculation formula having determined coefficients;
the step that a value of work by the user on the elastic mat at each detection point of the elastic mat is determined according to the deformation degree and the deformation duration of each preset detection point of the surface of the elastic mat and a calculation formula having determined coefficients and corresponding to each detection point may include that:
   a current exercise type is acquired;
   a calculating formula having determined coefficients and corresponding to each detection point under the current exercise type is determined according to a relationship among the exercise type, detection points and a calculation formula having determined coefficients; and
   a value of work by the user on the elastic mat at each detection point of the elastic mat is determined according to the deformation degree and the deformation duration of each preset detection point of the surface of the elastic mat and a calculation formula having determined coefficients and corresponding to each detection point under the current exercise type.

In this way, a value of work by the user performing each type of exercise on the elastic mat may be calculated accurately based on a corresponding calculation formula.

According to an exemplary embodiment, the step that the exercise type is acquired may include that:
the current exercise type is determined according to the deformation information of the surface of the elastic mat; or,
the current exercise type set by the user is acquired.

According to an exemplary embodiment, the method may further include that:
values of energy acquired for many times are accumulated, and when an accumulated value reaches a preset threshold, a prompt signal is sent out.

In this way, the user may know in time when a value of energy consumed via an exercise reaches a target value every time, thus reasonably and effectively making subsequent arrangement.

According to an exemplary embodiment, after the value of energy is recorded, the method may further include that:
the value of energy is transmitted to a bound target terminal so as to enable the target terminal to make statistics and display the value of energy.

In this way, the user may query a history record of a value of energy consumed via an exercise on the terminal more conveniently.

According to a second aspect of the embodiments of the present invention, a device for determining a value of consumed energy is provided in accordance with claim 7.

The advantages and technical effects of the device according to the invention correspond to those of the method presented above.

According to a particular embodiment, the device may further include:
a prompting module, configured to accumulate energy values acquired for many times, and when an accumulated value reaches a preset threshold, send out a prompt signal; and/or a transmitting module, configured to transmit the energy value to a bound target terminal so as to enable the target terminal to make statistics and display the energy value.

According to a third aspect of the embodiments of the present invention, a device for determining a value of consumed energy is provided. The device includes:
a processor; and
a memory for storing instructions executable by the processor,
wherein the processor is configured to:
   acquire deformation information of the surface of an elastic mat;
   determine, according to the deformation information of the surface of the elastic mat, a value of energy consumed by a user when corresponding deformation is generated; and record the value of energy.

In one particular embodiment, the steps of the method for determining a value of consumed energy are determined by computer program instructions.

Consequently, according to a seventh aspect, the invention is also directed to a computer program for executing the steps of the method for determining a value of consumed energy as described above when this program is executed by a computer.

This program can use any programming language and take the form of source code, object code or a code intermediate between source code and object code, such as a partially compiled form, or any other desirable form.

The invention is also directed to a computer-readable information medium containing instructions of a computer program as described above.

The information medium can be any entity or device capable of storing the program. For example, the support can include storage means such as a ROM, for example a CD ROM or a microelectronic circuit ROM, or magnetic storage means, for example a diskette (floppy disk) or a hard disk.

Alternatively, the information medium can be an integrated circuit in which the program is incorporated, the circuit being adapted to execute the method in question or to be used in its execution.

It is to be understood that both the foregoing general description and the following detailed description are exemplary and explanatory only and are not restrictive of the present invention.

### BRIEF DESCRIPTION OF THE DRAWINGS

The accompanying drawings, which are incorporated into and constitute a part of this specification, illustrate embodiments consistent with the embodiments of the present invention and, together with the description, serve to explain the principles of the embodiments of the present invention, in which:
Fig. 1 is a flowchart showing a method for determining a value of consumed energy according to an exemplary embodiment;
Fig. 2 is a schematic diagram illustrating an exercise on an elastic mat according to an exemplary embodiment;
Fig. 3a and Fig. 3b are diagrams illustrating an initial position according to an exemplary embodiment;
Fig. 4a and Fig. 4b are diagrams illustrating a maximum deformation position according to an exemplary embodiment;
Fig. 5 is a flowchart showing a method for determining a value of consumed energy according to an exemplary embodiment;
Fig. 6a and Fig. 6b are schematic diagrams illustrating actual detection according to an exemplary embodiment;
Fig. 7 is a structural diagram illustrating a device for determining a value of consumed energy according to an exemplary embodiment;
Fig. 8 is a structural diagram illustrating a device for determining a value of consumed energy according to an exemplary embodiment;
Fig. 9 is a structural diagram illustrating a device for determining a value of consumed energy according to an exemplary embodiment;
Fig. 10 is a structural diagram illustrating a device for determining a value of consumed energy according to an exemplary embodiment;
Fig. 11 is a structural diagram illustrating a device for determining a value of consumed energy according to an exemplary embodiment; and
Fig. 12 is a structural diagram illustrating an elastic mat according to an exemplary embodiment.

### DETAILED DESCRIPTION

Reference will now be made in detail to exemplary embodiments, examples of which are illustrated in the accompanying drawings. The following description refers to the accompanying drawings in which the same numbers in different drawings represent the same or similar elements unless otherwise represented. The implementations set forth in the following description of exemplary embodiments do not represent all implementations consistent with the invention. Instead, they are merely examples of apparatuses and methods consistent with aspects related to the invention as recited in the appended claims.

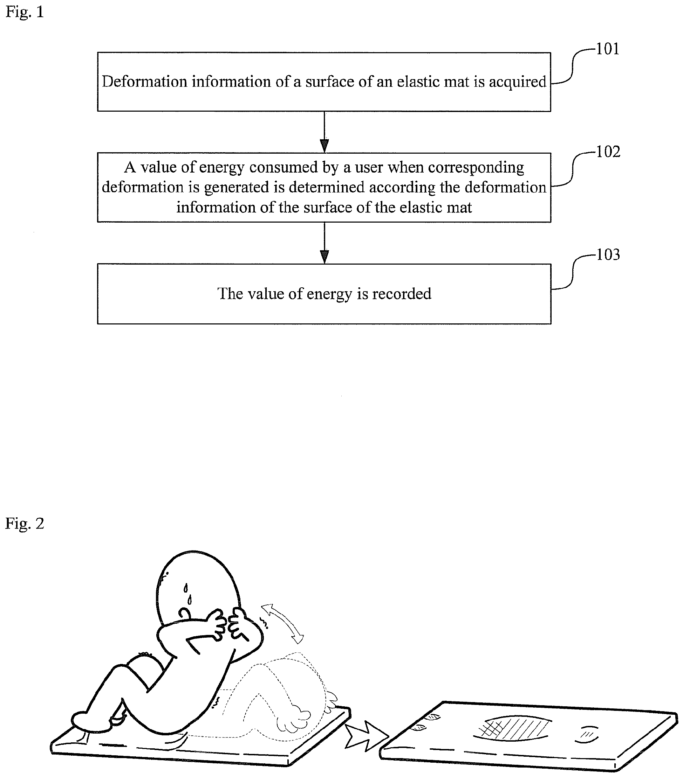

An exemplary embodiment of the present invention provides a method for determining a value of consumed energy. As shown in Fig. 1, the method may include the following steps.
Step 101: Deformation information of the surface of an elastic mat is acquired.
Step 102: A value of energy consumed by a user when corresponding deformation is generated is determined according to the deformation information of the surface of the elastic mat.
Step 103: The value of energy is recorded.

In an embodiment of the present invention, deformation information of the surface of an elastic mat is acquired; a value of energy consumed by a user when corresponding deformation is generated is determined according to the deformation information of the surface of the elastic mat; and the value of energy is recorded. Thus, the user will press the surface of the elastic mat in different degrees accordingly when doing exercises on the elastic mat, and the elastic mat can specifically determine, according to deformation information of the surface of the elastic mat, a value of energy consumed by the user when corresponding deformation is generated, so as to improve the accuracy in calculating a value of energy consumed via an exercise.

Another exemplary embodiment of the present invention provides a method for determining a value of consumed energy. The method may be applied in an elastic mat, wherein the elastic mat may be an intelligent mattress, an intelligent yoga mat and so on. A sensor, a processor and a memory may be arranged in the elastic mat. The sensor may be configured to detect deformation information of the surface of the elastic mat, and may be a displacement sensor, for example. The processor may be configured to process determination of a value of consumed energy. The memory may be configured to store data required and generated in the following processing. A communication component, a prompt component and so on may be further arranged in the elastic mat. The communication component may be configured to transmit data. The prompt component may be configured to send out a prompt signal, and may be a loudspeaker, an indicator lamp, a display screen and so on. In the present embodiment, a solution will be expounded by illustrating the elastic mat which is an intelligent mattress as an example. Other situations are similar to the solution, and will not be described repeatedly in the present embodiment. The processing as shown in Fig. 1 will be expounded below in conjunction with embodiments, and the content may be as follows.

Step 101: Deformation information of the surface of the elastic mat is acquired.

During implementation, when a user does exercises on the elastic mat, the elastic surface of the elastic mat may be pressed by the user to deform accordingly, as shown in Fig. 2. At the moment, the sensor in the elastic mat may detect and record deformation information of the surface of the elastic mat.

Optionally, the deformation information may include a deformation degree and a deformation duration of each preset detection point of the surface of the elastic mat, wherein the deformation degree is the distance from an initial position to a maximum deformation position of the surface of the elastic mat at the detection point during one deformation process; and the deformation duration is a duration in which the surface of the elastic mat is deformed from the initial position to the maximum deformation position at the detection point during one deformation process. The initial position refers to a position where the surface of the elastic mat locates at the detection point after the surface of the elastic mat is deformed and stabilized when the user is on the surface of the elastic mat and is ready to do exercises. One deformation process may be one of the cyclic change processes in which the amount of deformation of the surface of the elastic mat increases and then decreases constantly.

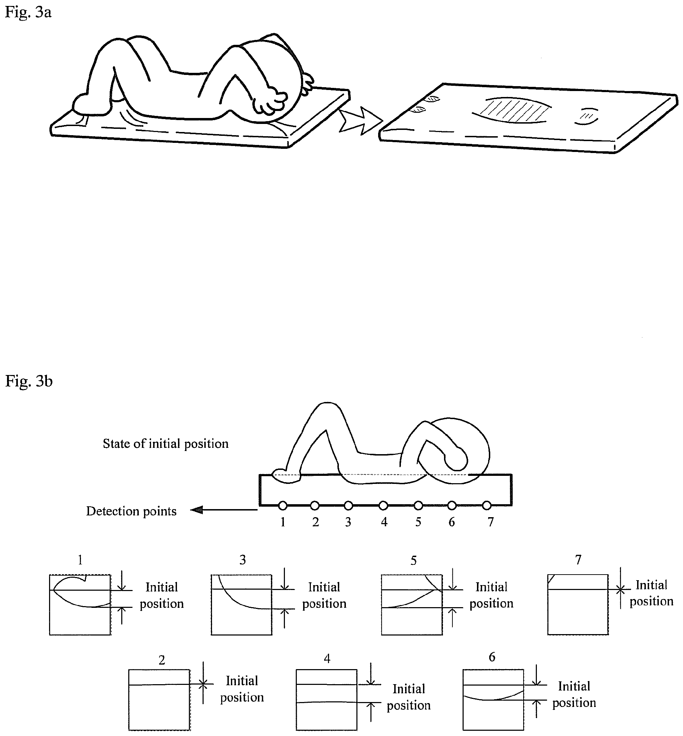

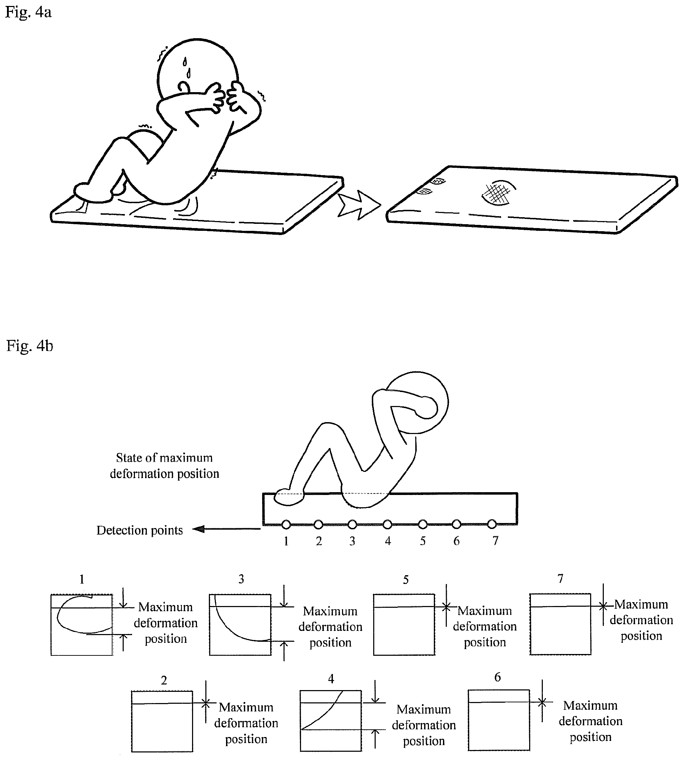

During implementation, when the user does exercises on the elastic mat, the user may press the elastic mat to deform the surface of the elastic mat accordingly. At the moment, a displacement senor on the elastic mat may detect a displacement (i.e. a deformation degree) corresponding to each detection point of the elastic mat in the vertical direction of the surface of the elastic mat and a period of time (i.e. a deformation duration) used to generate the corresponding displacement. To facilitate understanding, an example that the user does sit-ups on the elastic mat is illustrated here. First, the user may lie on his/her back on the elastic mat and presses the surface of the elastic mat to a certain degrees under the action of gravity. When the elastic mat is in a stable state after the user lies down, a pressing degree of the surface of the elastic mat at each detection point may be recorded as an initial position, as shown in Fig. 3a and Fig. 3b. Subsequently, the user begins to lift the upper half of the body to a sitting state. Then the user lies down again to recover to a lying state, and at the moment, the surface of the elastic mat recovers to the initial position again, as shown in Fig. 4a and Fig. 4b. The process above may be recorded as one deformation process. A pressing degree of the surface of the elastic mat at each detection point may be recorded in real time during the process, and a position with the maximum pressing degree on the surface of the elastic mat is recorded as a maximum deformation position. For each detection point, the deformation degree may be the distance from an initial position to a maximum deformation position of the surface corresponding to the detection point of the elastic mat during the one deformation process, while the deformation duration may be a duration in which the surface corresponding to the detection point of the elastic mat is deformed from the initial position to the maximum deformation position during the one deformation process.

Step 102: A value of energy consumed by a user when corresponding deformation is generated is determined according to the deformation information of the surface of the elastic mat.

During implementation, the elastic mat may determine, according to the sensor-recorded deformation information of the surface of the elastic mat and a preset method for determining a value of energy, a value of energy consumed when the user does the exercise corresponding to the deformation.

Optionally, the processing of Step 102 may be as follows for the situation that the deformation information includes a deformation degree and a deformation duration of each preset detection point of the surface of the elastic mat: a value of work by the user on the elastic mat at each detection point of the elastic mat is determined according to the deformation degree and the deformation duration of each preset detection point of the surface of the elastic mat; and the sum of values of work corresponding to all preset detection points is determined to be the value of the energy consumed by the user.

During implementation, after determining a deformation degree and a deformation duration at each preset detection point, the elastic mat may determine, according to the deformation degree and the deformation duration of each preset detection point of the surface of the elastic mat and a preset method for determining a value of energy, a value of work by the user on the elastic mat at each detection point, and then selects values of work corresponding to preset detection points, and determines the sum of values of work corresponding to all preset detection points to be the value of the energy consumed when the user does the exercise corresponding to the deformation.

Step 103: The value of energy is recorded.

During implementation, the elastic mat may record the determined value of the energy consumed by the user in a memory.

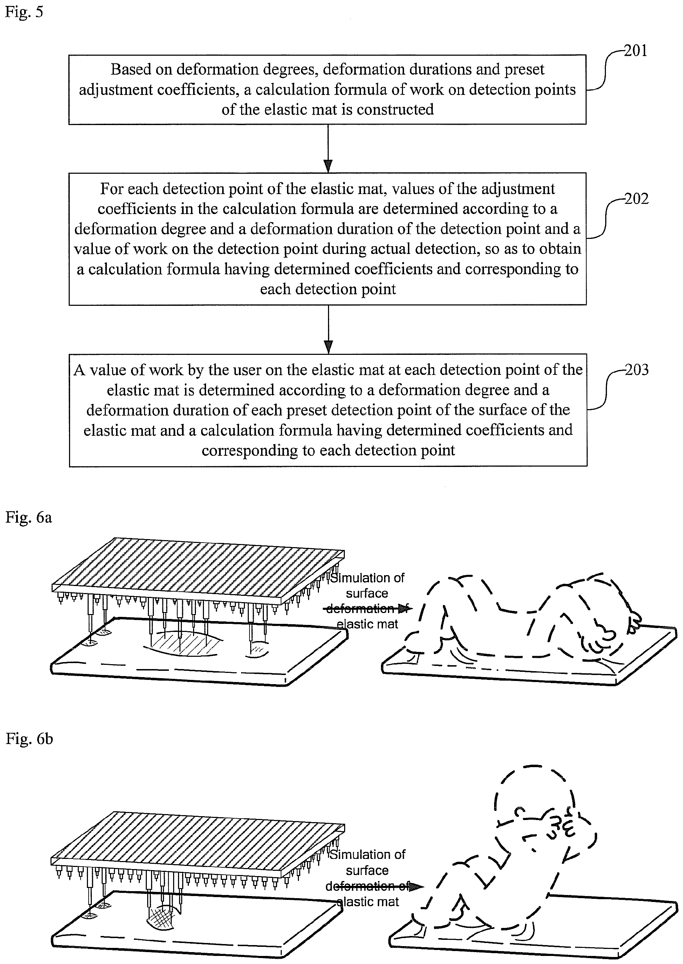

In an embodiment of the present invention, a calculation formula may be preset to calculate the value of energy according to the deformation information. As shown in Fig. 5, the processing may include the following steps.

Step 201: Based on deformation degrees, deformation durations and preset adjustment coefficients, a calculation formula of work on detection points of the elastic mat is constructed.

During implementation, a technician may research and determine various variables that affect work on detection points of the elastic mat, such as a deformation degree and a deformation duration of the surface of the elastic mat and other factors, thus constructing a calculation formula of work on the detection points of the elastic mat based on these variables.

Step 202: For each detection point of the elastic mat, values of the adjustment coefficients in the calculation formula are determined according to the deformation degree and the deformation duration of the detection point and a value of work on the detection point during actual detection, so as to obtain a calculation formula having determined coefficients and corresponding to each detection point.

During implementation, a technician may do exercises on the elastic mat during actual detection, such as sit-ups, leg lifts and so on. At the moment, the elastic mat will record a deformation degree and a deformation duration of the surface corresponding to each detection point of the elastic mat. In the meanwhile, a value of energy consumed when the technical does exercises may be detected by a precise detection instrument. After the exercise, the exercise performed by the technician may be simulated by a plurality of machines for exercise simulation. In other words, each machine presses the surface corresponding to a detection point of the elastic mat according to the deformation degree and a deformation duration recorded by the elastic mat, so as to simulate the exercise, as shown in Fig. 6a and Fig. 6b. After the simulation, each machine may calculate, according to the value of energy consumed by the machine and a mechanical efficiency, a value of work by the machine on the detection point. Subsequently, values of work by all machines on the detection points may be accumulated to obtain a total value of the work by all machines on the elastic mat during the simulation process, thus acquiring a relationship between the work by the machines on the elastic mat and work by the technician on the elastic mat according to the total value of the work by the machines on the elastic mat and the value of the energy consumed by the technician when doing exercises. Subsequently, work by the technician on each detection point during the exercise on the elastic mat may be acquired with reference to a value of work by each machine on a corresponding detection point. In this way, values of the adjustment coefficients in the calculation formula may be determined by means of statistical fit after performing the actual test for many times, thereby acquiring a calculation formula having determined coefficients and corresponding to each detection point.

Optionally, different exercise types may correspond to different calculation formulae, and the processing of Step 202 may be as follows: values of the adjustment coefficients in the calculation formula are determined for each detection point of the elastic mat according to the deformation degree and a deformation duration of the detection point and a value of work on the detection point during actual detection under each exercise type, so as to obtain a relationship among the exercise type, the detection point and a calculation formula having determined coefficients.

During implementation, a technician may do an exercise of a preset type on the elastic mat, such as sit-ups, leg lifts and so on. At the moment, the elastic mat will record a deformation degree and a deformation duration of the surface corresponding to each detection point of the elastic mat. In the meanwhile, a value of energy consumed by the technician when doing an exercise of a certain preset type may be detected by a precise detection instrument. After the exercise, the exercise done by the technician may be simulated by a plurality of machines for exercise simulation. In other words, each machine presses the surface corresponding to a detection point of the elastic mat according to the deformation degree and the deformation duration recorded by the elastic mat, so as to simulate the exercise. After the simulation, each machine may calculate, according to a value of energy consumed by the machine and a mechanical efficiency, a value of work by the machine on the detection point. Subsequently, values of work by all machines on the detection points may be accumulated to obtain a total value of the work by all machines on the elastic mat during the simulation process, thus acquiring a relationship between the work by the machines on the elastic mat and work by the technician on the elastic mat according to the total value of the work by the machines on the elastic mat and the value of the energy consumed by the technician when doing the exercise. Subsequently, work by the technician on each detection point during the exercise of the preset type on the elastic mat may be acquired with reference to a value of work by each machine on a corresponding detection point. In this way, values of the adjustment coefficients in the calculation formula may be determined by means of statistical fit after performing the actual test for many times, thereby obtaining a relationship among the exercise type, the detection point and a calculation formula having determined coefficients.

Step 203: A value of work by the user on the elastic mat at each detection point of the elastic mat is determined according to the deformation degree and the deformation duration of each preset detection point of the surface of the elastic mat and a calculation formula having determined coefficients and corresponding to each detection point.

During implementation, after determining a deformation degree and a deformation duration at each preset detection point, the elastic mat may determine a value of work by the user on the elastic mat at each detection point according to the deformation degree and the deformation duration of each preset detection point of the surface of the elastic mat and a calculation formula having determined coefficients and corresponding to each detection point, and then selects values of work corresponding to preset detection points, and determines the sum of values of work corresponding to all preset detection points to be the value of the energy consumed when the user does the exercise corresponding to the deformation.

Optionally, for the calculation corresponding to the exercise type, the processing of Step 203 may be as follows: a current exercise type is acquired; a calculating formula having determined coefficients and corresponding to each detection point under the current exercise type is determined according to a relationship among the exercise type, detection points and a calculation formula having determined coefficients; a value of work by the user on the elastic mat at each detection point of the elastic mat is determined according to the deformation degree and the deformation duration of each preset detection point of the surface of the elastic mat and a calculation formula having determined coefficients and corresponding to each detection point under the current exercise type.

During implementation, the elastic mat may acquire the current exercise type according to a preset method, and then determines, according to the relationship among the exercise type, the detection points and the calculation formula having the determined coefficients, which is acquired in Step 203, a calculating formula having determined coefficients and corresponding to each detection point under the current exercise type, thus after a deformation degree and a deformation duration at each preset detection point are determined, a value of work by the user on the elastic mat at each detection point may be determined according to the deformation degree and the deformation duration of each preset detection point of the surface of the elastic mat and a calculation formula having determined coefficients and corresponding to each detection point, and then values of work corresponding to preset detection points may be selected and the sum of values of work corresponding to all preset detection points is determined to be a value of energy consumed by the user when doing the exercise corresponding to the deformation.

It is worth mentioning that, similar to the situation of the exercise type above, calculation formulae may be determined through actual detection for elastic mats with different total use time, and elastic mats made of different materials, thus the user can specifically select a corresponding calculation formula during a process of exercising on an elastic mat.

There may be elastic mats of different types for users having different heights and weights, and an optimal use area of an elastic mat in an exercise is described in a manual of the elastic mat. More vividly, an outline may be marked on the elastic mat, and a detected value of energy consumed via an exercise is the most accurate when the user does exercises in an area defined by the outline.

Optionally, there are various approaches for acquiring the current exercise type, and two feasible approaches are provided as follows.

The first approach includes that the current exercise type is determined according to the deformation information of the surface of the elastic mat.

During implementation, different exercise type may correspond to specific deformation information of the surface of the elastic mat, thus the elastic mat can determine, according to detected deformation information of the surface, an exercise type closest to the deformation information so as to determine the current exercise type.

The second approach includes that the current exercise type set by the user is acquired.

During implementation, the user may set the current exercise type on a functional interface of the elastic mat or on a terminal bound with the elastic mat, thus the elastic mat can acquire the current exercise type according to the content set by the user.

Optionally, the elastic mat may send out a prompt signal when the amount of an exercise of the user reaches a target value, and corresponding processing may be as follows: values of energy acquired for many times are accumulated, and when an accumulated value reaches a preset threshold, a prompt signal is sent out.

During implementation, the user may preset a threshold for a value of energy consumed by each exercise according to body conditions and so on. When receiving an exercise start instruction inputted by the user, the elastic mat resets a previously-recorded value of energy consumed by the user, and records a value of energy consumed by the user during a single deformation. Subsequently, the elastic mat accumulates values of energy consumed by the user, which are acquired for many times, and may send out a prompt signal when an accumulated value reaches the preset threshold. For example, an indicator lamp flashes or a prompt ring sounds so as to prompt the user that a value of energy consumed by a current exercise has reached a target value or is too high, and the user should pay attention to rest in time.

Optionally, the elastic mat may transmit the energy value to a bound target terminal after the user finishes the exercise, so as to enable the target terminal to make statistics and display the energy value.

In an embodiment of the present invention, deformation information of the surface of an elastic mat is acquired; a value of energy consumed by a user when corresponding deformation is generated is determined according to the deformation information of the surface of the elastic mat; and the value of energy is recorded. Thus, the user will press the surface of the elastic mat in different degrees accordingly when doing exercises on the elastic mat, and the elastic mat can specifically determine, according to deformation information of the surface of the elastic mat, a value of energy consumed by the user when corresponding deformation is generated, so as to improve the accuracy in calculating a value of energy consumed via an exercise.

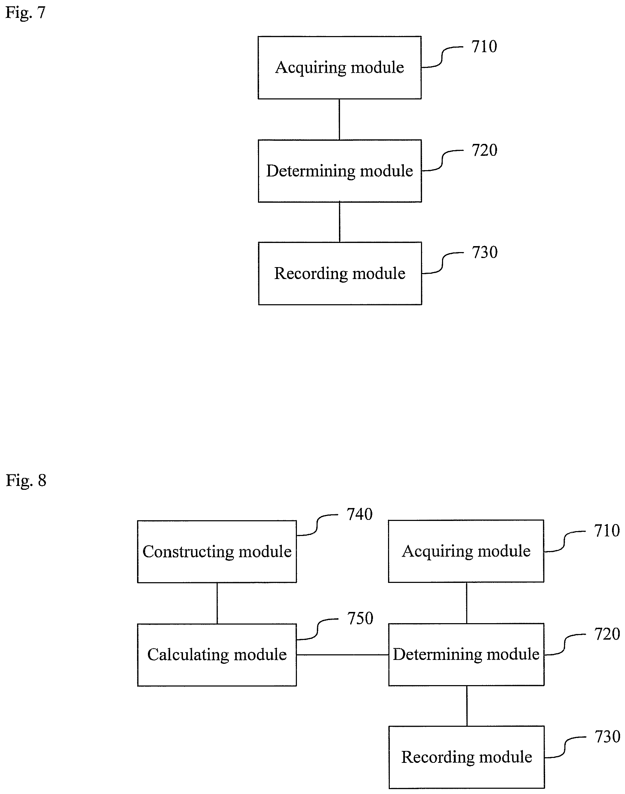

Based on the same technical conception, another exemplary embodiment of the present invention further provides a device for determining a value of consumed energy. As shown in Fig. 7, the device includes an acquiring module 710, a determining module 720 and a recording module 730.

The acquiring module 710 is configured to acquire deformation information of the surface of an elastic mat.

The determining module 720 is configured to determine, according the deformation information of the surface of the elastic mat, a value of energy consumed by a user when corresponding deformation is generated.

The recording module 730 is configured to record the value of energy.

Optionally, the deformation information includes a deformation degree and a deformation duration of each preset detection point of the surface of the elastic mat,
wherein the deformation degree is the distance from an initial position to a maximum deformation position of the surface of the elastic mat at the detection point during one deformation process; and the deformation duration is a duration in which the surface of the elastic mat is deformed from the initial position to the maximum deformation position at the detection point during one deformation process.

Optionally, the determining module 720 is configured to:
determine, according to the deformation degree and the deformation duration of each preset detection point of the surface of the elastic mat, a value of work by the user on the elastic mat at each detection point of the elastic mat; and
determine the sum of values of work corresponding to all preset detection points to be the value of the energy consumed by the user.

Optionally, as shown in Fig. 8, the device further includes:
a constructing module 740, configured to construct a calculation formula of work on detection points of the elastic mat based on deformation degrees, deformation durations and preset adjustment coefficients;
a calculating module 750, configured to determine, for each detection point of the elastic mat, values of the adjustment coefficients in the calculation formula according to the deformation degree and the deformation duration of the detection point and a value of work on the detection point during actual detection, so as to obtain a calculation formula having determined coefficients and corresponding to each detection point.

The determining module 720 is configured to:
determine, according to the deformation degree and the deformation duration of each preset detection point of the surface of the elastic mat and a calculation formula having determined coefficients and corresponding to each detection point, a value of work by the user on the elastic mat at each detection point of the elastic mat.

Optionally, the calculating module 750 is configured to:
determine, for each detection point of the elastic mat, values of the adjustment coefficients in the calculation formula, according to the deformation degree and the deformation duration of the detection point and a value of work on the detection point during actual detection under each exercise type, so as to obtain a relationship among the exercise type, the detection point and a calculation formula having determined coefficients.

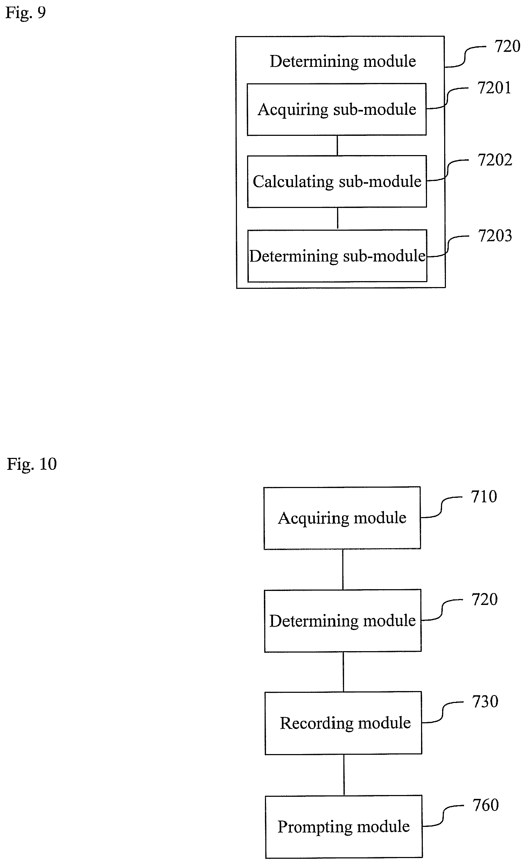

As shown in Fig. 9, the determining module 720 includes:
an acquiring sub-module 7201, configured to acquire a current exercise type;
a calculating sub-module 7202 configured to, according to a relationship among the exercise type, detection points and a calculation formula having determined coefficients, determine a calculating formula having determined coefficients and corresponding to each detection point under the current exercise type; and
a determining sub-module 7203, configured to determine, according to the deformation degree and the deformation duration of each preset detection point of the surface of the elastic mat and a calculation formula having determined coefficients and corresponding to each detection point under the current exercise type, a value of work by the user on the elastic mat at each detection point of the elastic mat.

Optionally, the acquiring sub-module 7201 is configured to:
determine the current exercise type according to the deformation information of the surface of the elastic mat; or,
acquire the current exercise type set by the user.

Optionally, as shown in Fig. 10, the device further includes:
a prompting module 760, configured to accumulate energy values acquired for many times, and when an accumulated value reaches a preset threshold, send out a prompt signal.

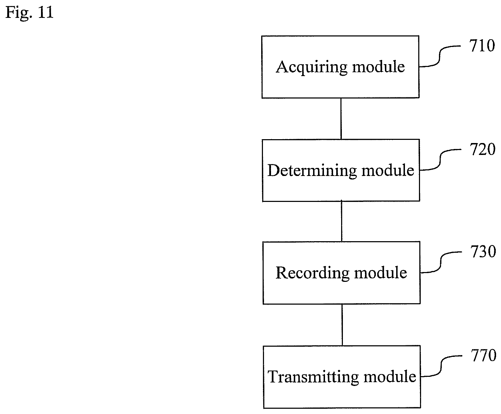

Optionally, as shown in Fig. 11, the device further includes:
a transmitting module 770, configured to transmit the energy value to a bound target terminal so as to enable the target terminal to make statistics and display the energy value.

A specific method for executing an operation by each module in the device in the above embodiment has been expounded in the embodiment related to the method, and will not be elaborated and described in details herein.

In an embodiment of the present invention, deformation information of the surface of an elastic mat is acquired; a value of energy consumed by a user when corresponding deformation is generated is determined according to the deformation information of the surface of the elastic mat; and the value of energy is recorded. Thus, the user will press the surface of the elastic mat in different degrees accordingly when doing exercises on the elastic mat, and the elastic mat can specifically determine, according to deformation information of the surface of the elastic mat, a value of energy consumed by the user when corresponding deformation is generated, so as to improve the accuracy in calculating a value of energy consumed via an exercise.

It should be noted that only division of the functional modules above is illustrated as an example when the devices for determining a value of consumed energy according to the above embodiments determine a value of consumed energy. During practical application, the functions may be completed by different modules as required. In other words, the internal structures of the devices are divided into different functional modules so as to complete all or some functions described above. Besides, the devices for determining a value of consumed energy according to the above embodiments and the embodiments of the method for determining a value of consumed energy belong to the same conception. Details of specific implementation processes of the devices for determining a value of consumed energy may refer to the embodiments related to the methods, and will not be repeated here.

Another exemplary embodiment of the present invention also shows a structural diagram of an elastic mat. The elastic mat may be an intelligent mattress and so on.

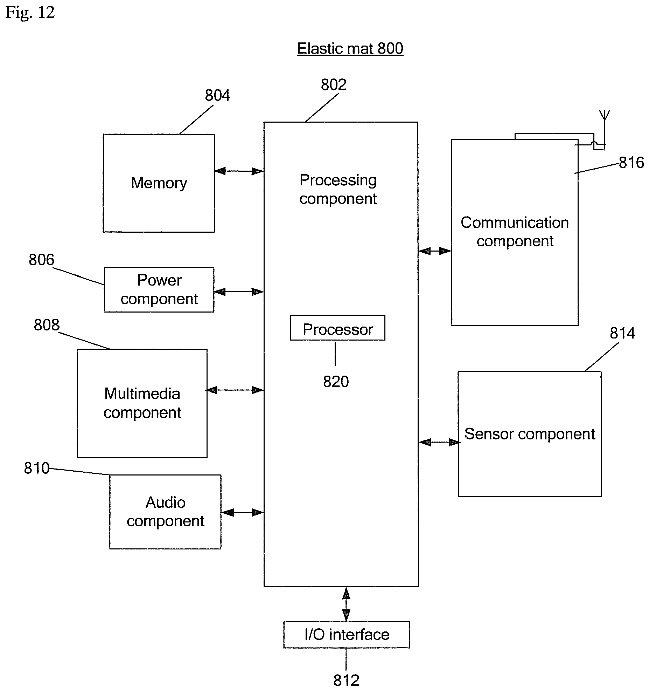

Referring to Fig. 12, the elastic mat 800 may include one or more following components: a processing component 802, a memory 804, a power component 806, a multimedia component 808, an audio component 810, an Input/Output (I/O) interface 812, a sensor component 814 and a communication component 816.

Generally, the processing component 802 controls overall operations of the elastic mat 800, such as operations associated with displaying and recording operations. The processing component 802 may include one or more processors 820 to execute instructions so as to complete all or part of the steps of the abovementioned methods. Moreover, the processing component 802 may include one or more modules which facilitate interaction between the processing component 802 and other components. For example, the processing component 802 may include a multimedia module so as to facilitate interaction between the multimedia component 808 and the processing component 802.

The memory 804 is configured to store various types of data to support the operations in the elastic mat 800. Examples of these data include instructions for any applications or methods operated on the elastic mat 800 and so on. The memory 804 may be implemented by a volatile or non-volatile storage device of any type or a combination thereof, such as a Static Random-Access Memory (SRAM), an Electrically Erasable Programmable Read-Only Memory (EEPROM), an Erasable Programmable Read-Only Memory (EPROM), a Programmable Read-Only Memory (PROM), a Read-Only Memory (ROM), a magnetic memory, a flash memory, a magnetic disk or an optical disk.

The power component 806 provides power for various components of the elastic mat 800. The power component 806 may include a power source management system, one or more power supplies, and other components associated with generation, management and distribution of power for the elastic mat 800.

The multimedia component 808 includes a screen providing an output interface between the elastic mat 800 and a user. In some embodiments, the screen may include a Liquid Crystal Display (LCD) and a Touch Panel (TP). If the screen includes the TP, the screen may be implemented as a touch screen to receive an input signal from the user. The touch panel includes one or more touch sensors to sense touches, swipes and gestures on the TP. The touch sensor may not only sense a boundary of a touch or swipe action, but also detect a duration and a pressure related to the touch or swipe action. In some embodiments, the multimedia component 808 includes a front camera and/or a rear camera. When the elastic mat 800 is in an operation mode, such as a camera mode or a video mode, the front camera and/or the rear camera may receive external multimedia data. Each front camera and each rear camera may be a fixed optical lens system or may be provided with focusing and optical zooming capabilities.

The audio component 810 is configured to output and/or input an audio signal. For example, the audio component 810 includes a Microphone (MIC). When the elastic mat 800 is in an operation mode, such as a playing mode, a recording mode and a voice recognition mode, the MIC is configured to receive an external audio signal. The received audio signal may be further stored in the memory 804 or sent by the communication component 816.

The I/O interface 812 provides an interface between the processing component 802 and a peripheral interface module. The peripheral interface module may be a keyboard, a click wheel, a button and so on. The button may include, but not limited to a home button, a volume button, a starting button and a locking button.

The sensor component 814 includes one or more sensors configured to provide status assessment in various aspects for the elastic mat 800. For example, the sensor component 814 may detect an on/off state of the elastic mat 800, and relative positioning of the components, such as a display and a keypad of the elastic mat 800. The sensor component 814 may further detect a change in a position of the elastic mat 800 or a component of the elastic mat 800, presence or absence of a contact between the user and the elastic mat 800, orientation or acceleration/deceleration of the elastic mat 800 and a change in temperature of the elastic mat 800. The sensor component 814 may include a proximity sensor configured to detect presence of an object nearby without any physical contact. The sensor component 814 may further include an optical sensor, such as a Complementary Metal Oxide Semiconductor (CMOS) or Charge Coupled Device (CCD) image sensor used in an imaging application. In some embodiments, the sensor component 814 may further include an acceleration sensor, a gyroscope sensor, a magnetic sensor, a pressure sensor or a temperature sensor.

The communication component 816 is configured to facilitate wired or wireless communication between the elastic mat 800 and other devices. The elastic mat 800 may access a wireless network based on a communication standard, such as a Wireless Fidelity (WiFi) network, the second Generation (2G) or the third Generation (3G) network, or a combination thereof. In an exemplary embodiment, the communication component 816 receives a broadcast signal from an external broadcast management system or broadcasts related information through a broadcast channel. In an exemplary embodiment, the communication component 816 further includes a Near Field Communication (NFC) module to facilitate short-range communication. For example, the NFC module may be implemented based on a Radio-frequency Identification (RFID) technology, an Infrared Data Association (IrDA) technology, an Ultra Wide Band (UWB) technology, a Bluetooth (BT) technology and other technologies.

In an exemplary embodiment, the elastic mat 800 may be implemented by one or more Application Specific Integrated Circuits (ASICs), Digital Signal Processors (DSPs), Digital Signal Processing Devices (DSPDs), Programmable Logic Devices (PLDs), Field Programmable Gate Arrays (FPGAs), controllers, microcontrollers, microprocessors, or other electronic components, so as to execute the abovementioned method.

In the exemplary embodiment, there is also provided a non-temporary computer readable storage medium including instructions, such as a memory 804 including instructions. The instructions may be executed by a processor 820 of an elastic mat 800 to implement the above-described methods. For example, the non-temporary computer readable storage medium may be a ROM, a Compact Disc-ROM (CD-ROM), a magnetic tape, a floppy disk, an optical data memory and so on.

A non-temporary computer readable storage medium enables an elastic mat to execute a method for determining a value of consumed energy when instructions in the storage medium are executed by a processor of the elastic mat. The method includes that:
deformation information of the surface of an elastic mat is acquired;
a value of energy consumed by a user when corresponding deformation is generated is determined according to the deformation information of the surface of the elastic mat; and
the value of energy is recorded.

Optionally, the deformation information includes a deformation degree and a deformation duration of each preset detection point of the surface of the elastic mat,
wherein the deformation degree is the distance from an initial position to a maximum deformation position of the surface of the elastic mat at the detection point during one deformation process; and the deformation duration is a duration in which the surface of the elastic mat is deformed from the initial position to the maximum deformation position at the detection point during one deformation process.

Optionally, the step that the value of the energy consumed by the user when the corresponding deformation is generated is determined according to the deformation information of the surface of the elastic mat includes that:
a value of work of the user on the elastic mat at each detection point of the elastic mat is determined according to the deformation degree and the deformation duration of each preset detection point of the surface of the elastic mat; and
the sum of values of work corresponding to all preset detection points is determined to be the value of the energy consumed by the user.

Optionally, the method further includes that:
based on deformation degrees, deformation durations and preset adjustment coefficients, a calculation formula of work on detection points of the elastic mat is constructed;
for each detection point of the elastic mat, values of the adjustment coefficients in the calculation formula are determined according to the deformation degree and the deformation duration of the detection point and a value of work on the detection point during actual detection, so as to obtain a calculation formula having determined coefficients and corresponding to each detection point;
the step that a value of work of the user on the elastic mat at each detection point of the elastic mat is determined according to the deformation degree and the deformation duration of each preset detection point of the surface of the elastic mat includes that:
   a value of work by the user on the elastic mat at each detection point of the elastic mat is determined according to the deformation degree and the deformation duration of each preset detection point of the surface of the elastic mat and a calculation formula having determined coefficients and corresponding to each detection point.

Optionally, the step that values of the adjustment coefficients in the calculation formula are determined for each detection point of the elastic mat according to the deformation degree and the deformation duration of the detection point and a value of work on the detection point during actual detection, so as to obtain a calculation formula having determined coefficients and corresponding to each detection point includes that:
values of the adjustment coefficients in the calculation formula are determined for each detection point of the elastic mat according to the deformation degree and the deformation duration of the detection point and a value of work on the detection point during actual detection under each exercise type, so as to obtain a relationship among the exercise type, the detection point and a calculation formula having determined coefficients;
the step that a value of work of the user on the elastic mat at each detection point of the elastic mat is determined according to the deformation degree and the deformation duration of each preset detection point of the surface of the elastic mat and a calculation formula having determined coefficients and corresponding to each detection point includes that:
   a current exercise type is acquired;
   a calculating formula having determined coefficients and corresponding to each detection point under the current exercise type is determined according to a relationship among the exercise type, detection points and a calculation formula having determined coefficients;
   a value of work by the user on the elastic mat at each detection point of the elastic mat is determined according to the deformation degree and the deformation duration of each preset detection point of the surface of the elastic mat and a calculation formula having determined coefficients and corresponding to each detection point under the current exercise type.

Optionally, the step that the exercise type is acquired includes that:
the current exercise type is determined according to the deformation information of the surface of the elastic mat; or,
the current exercise type set by the user is acquired.

Optionally, the method further includes that:
energy values acquired for many times are accumulated, and when an accumulated value reaches a preset threshold, a prompt signal is sent out.

Optionally, after recording the energy value, the method further includes that:
the energy value is transmitted to a bound target terminal so as to enable the target terminal to make statistics and display the energy value.

In an embodiment of the present invention, deformation information of the surface of an elastic mat is acquired; a value of energy consumed by a user when corresponding deformation is generated is determined according to the deformation information of the surface of the elastic mat; and the value of energy is recorded. Thus, the user will press the surface of the elastic mat in different degrees accordingly when doing exercises on the elastic mat, and the elastic mat can specifically determine, according to deformation information of the surface of the elastic mat, a value of energy consumed by the user when corresponding deformation is generated, so as to improve the accuracy in calculating a value of energy consumed via an exercise.

### Industrial Application

In the embodiments of the present invention, deformation information of the surface of an elastic mat is acquired; a value of energy consumed by a user when corresponding deformation is generated is determined according to the deformation information of the surface of the elastic mat; and the value of energy is recorded. Thus, the user will press the surface of the elastic mat in different degrees accordingly when doing exercises on the elastic mat, and the elastic mat can specifically determine, according to deformation information of the surface of the elastic mat, a value of energy consumed by the user when corresponding deformation is generated, so as to improve the accuracy in calculating a value of energy consumed via an exercise.

## Claims

1. A method for determining a value of consumed energy, comprising:
acquiring (101), by a processing component of an elastic mat, deformation information of a surface of the elastic mat;
determining (102), by the processing component of the elastic mat, according to the deformation information of the surface of the elastic mat, a value of energy consumed by a user when corresponding deformation is generated; and
recording (103), by the processing component of the elastic mat, the value of energy,
**characterized in that** the deformation information comprises a deformation degree and a deformation duration of each preset detection point of the surface of the elastic mat,
wherein the deformation degree is the distance from an initial position to a maximum deformation position of the surface of the elastic mat at the detection point during one deformation process; and the deformation duration is a duration in which the surface of the elastic mat at the detection point is deformed from the initial position to the maximum deformation position during one deformation process, and
wherein the step of determining (102), by the processing component of the elastic mat,, according to the deformation information of the surface of the elastic mat, the value of the energy consumed by the user when the corresponding deformation is generated comprises:
determining, according to the deformation degree and the deformation duration of each preset detection point of the surface of the elastic mat, a value of work by the user on the elastic mat at each detection point of the elastic mat; and
determining the sum of values of work corresponding to all preset detection points to be the value of the energy consumed by the user.

2. The method according to claim 1, wherein the method further comprises:
based on deformation degrees, deformation durations and preset adjustment coefficients, constructing (201) a calculation formula of work on detection points of the elastic mat;
determining (202), for each detection point of the elastic mat, values of the adjustment coefficients in the calculation formula according to the deformation degree and the deformation duration of the detection point and a value of work on the detection point during actual detection, so as to obtain a calculation formula having determined coefficients and corresponding to each detection point;
the step of determining, according to the deformation degree and the deformation duration of each preset detection point of the surface of the elastic mat, the value of work by the user on the elastic mat at each detection point of the elastic mat comprises:
determining (203), according to the deformation degree and the deformation duration of each preset detection point of the surface of the elastic mat and the calculation formula having determined coefficients and corresponding to each detection point, the value of work by the user on the elastic mat at each detection point of the elastic mat.

3. The method according to claim 2, wherein the step of determining, for each detection point of the elastic mat, values of the adjustment coefficients in the calculation formula according to the deformation degree and the deformation duration of the detection point and the value of work on the detection point during actual detection, so as to obtain the calculation formula having determined coefficients and corresponding to each detection point comprises:
determining, for each detection point of the elastic mat, values of the adjustment coefficients in the calculation formula, according to the deformation degree and the deformation duration of the detection point and a value of work on the detection point during actual detection under each exercise type, so as to obtain a relationship among the exercise type, the detection point and a calculation formula having determined coefficients;
the step of determining, according to the deformation degree and the deformation duration of each preset detection point of the surface of the elastic mat and the calculation formula having determined coefficients and corresponding to each detection point, the value of work by the user on the elastic mat at each detection point of the elastic mat comprises:
acquiring a current exercise type;
according to the relationship among the exercise type, detection points and the calculation formula having determined coefficients, determining the calculating formula having determined coefficients and corresponding to each detection point under the current exercise type; and
determining, according to the deformation degree and the deformation duration of each preset detection point of the surface of the elastic mat and the calculation formula having determined coefficients and corresponding to each detection point under the current exercise type, the value of work by the user on the elastic mat at each detection point of the elastic mat.

4. The method according to claim 3, wherein the step of acquiring the exercise type comprises:
determining, according to the deformation information of the surface of the elastic mat, the current exercise type; or,
acquiring the current exercise type set by the user.

5. The method according to claim 1, further comprising:
accumulating values of energy acquired for many times, and when an accumulated value reaches a preset threshold, sending out a prompt signal.

6. The method according to claim 1, wherein after recording the value of energy, the method further comprises:
transmitting the value of energy to a bound target terminal so as to enable the target terminal to make statistics and display the value of energy.

7. A device for determining a value of consumed energy, comprising:
an acquiring module (710), configured to acquire deformation information of a surface of an elastic mat;
a determining module (720), configured to determine, according to the deformation information of the surface of the elastic mat, a value of energy consumed by a user when corresponding deformation is generated; and
a recording module (730), configured to record the value of energy, wherein the deformation information comprises a deformation degree and a deformation duration of each preset detection point of the surface of the elastic mat,
**characterized in that** wherein the deformation degree is the distance from an initial position to a maximum deformation position of the surface of the elastic mat at the detection point during one deformation process; and the deformation duration is a duration in which the surface of the elastic mat at the detection point is deformed from the initial position to the maximum deformation position during one deformation process,
and wherein the determining module (720) is configured to:
determine, according to the deformation degree and the deformation duration of each preset detection point of the surface of the elastic mat, a value of work by the user on the elastic mat at each detection point of the elastic mat; and
determine the sum of values of work corresponding to all preset detection points to be the value of the energy consumed by the user.

8. The device according to claim 7, further comprising:
a prompting module (760), configured to accumulate values of energy acquired for many times, and when an accumulated value reaches a preset threshold, send out a prompt signal; and/or
a transmitting module (770), configured to transmit the value of energy to a bound target terminal so as to enable the target terminal to make statistics and display the value of energy.

9. A computer program including instructions for executing the steps of a method for determining a value of consumed energy according to any one of claims 1 to 6 when said program is executed by a computer.

10. A recording medium readable by a computer and having recorded thereon a computer program including instructions for executing the steps of a method for determining a value of consumed energy according to any one of claims 1 to 6.

## Patentansprüche

1. Verfahren zur Bestimmung eines Werts verbrauchter Energie, umfassend:
Beziehen (101) von Verformungsinformationen einer Oberfläche einer elastischen Matte durch eine Verarbeitungskomponente der elastischen Matte,
Bestimmen (102) eines Werts der Energie, die von einem Benutzer verbraucht wird, wenn eine entsprechende Verformung erzeugt wird, durch die Verarbeitungskomponente der elastischen Matte gemäß den Verformungsinformationen der Oberfläche der elastischen Matte, und
Aufzeichnen (103) des Werts der Energie durch die Verarbeitungskomponente der elastischen Matte,
**dadurch gekennzeichnet, dass** die Verformungsinformationen einen Verformungsgrad und eine Verformungsdauer von jedem voreingestellten Erfassungspunkt der Oberfläche der elastischen Matte umfassen,
wobei der Verformungsgrad die Entfernung von einer Anfangsposition zu einer maximalen Verformungsposition der Oberfläche der elastischen Matte an dem Erfassungspunkt während eines Verformungsvorgangs ist, und die Verformungsdauer eine Dauer ist, in der die Oberfläche der elastischen Matte an dem Erfassungspunkt aus der Anfangsposition zu der maximalen Verformungsposition während eines Verformungsvorgangs verformt wird, und
wobei der Schritt des Bestimmens (102) des Werts der Energie, die von dem Benutzer verbraucht wird, wenn die entsprechenden Verformungsinformationen erzeugt werden, durch die Verarbeitungskomponente der elastischen Matte gemäß den Verformungsinformationen der Oberfläche der elastischen Matte umfasst:
Bestimmen eines Werts von Arbeit durch den Benutzer auf der elastischen Matte an jedem Erfassungspunkt der elastischen Matte gemäß dem Verformungsgrad und der Verformungsdauer von jedem voreingestellten Erfassungspunkt der Oberfläche der elastischen Matte, und
Bestimmen der Summe von Werten von Arbeit, die allen voreingestellten Erfassungspunkten entsprechen, als der Wert der Energie, die von dem Benutzer verbraucht wird.

2. Verfahren nach Anspruch 1, wobei das Verfahren ferner umfasst:
Konstruieren (201) einer Berechnungsformel von Arbeit an Erfassungspunkten der elastischen Matte auf der Basis von Verformungsgraden, Verformungsdauern und voreingestellten Einstellungskoeffizienten,
Bestimmen (202), für jeden Erfassungspunkt der elastischen Matte, von Werten der Einstellungskoeffizienten in der Berechnungsformel gemäß dem Verformungsgrad und der Verformungsdauer des Erfassungspunkts und eines Werts von Arbeit an dem Erfassungspunkt während einer tatsächlichen Erfassung, um eine Berechnungsformel zu erhalten, die bestimmte Koeffizienten aufweist und jedem Erfassungspunkt entspricht,
wobei der Schritt des Bestimmens des Werts von Arbeit durch den Benutzer auf der elastischen Matte an jedem Erfassungspunkt der elastischen Matte gemäß dem Verformungsgrad und der Verformungsdauer von jedem voreingestellten Erfassungspunkt der Oberfläche der elastischen Matte umfasst:
Bestimmen (203) des Werts von Arbeit durch den Benutzer auf der elastischen Matte an jedem Erfassungspunkt der elastischen Matte gemäß dem Verformungsgrad und der Verformungsdauer von jedem voreingestellten Erfassungspunkt der Oberfläche der elastischen Matte und der Berechnungsformel, die bestimmte Koeffizienten aufweist und jedem Erfassungspunkt entspricht.

3. Verfahren nach Anspruch 2, wobei der Schritt des Bestimmens, für jeden Erfassungspunkt der elastischen Matte, von Werten der Einstellungskoeffizienten in der Berechnungsformel gemäß dem Verformungsgrad und der Verformungsdauer des Erfassungspunkts und des Werts von Arbeit an dem Erfassungspunkt während einer tatsächlichen Erfassung, um die Berechnungsformel zu erhalten, die bestimmte Koeffizienten aufweist und jedem Erfassungspunkt entspricht, umfasst:
Bestimmen, für jeden Erfassungspunkt der elastischen Matte, von Werten der Einstellungskoeffizienten in der Berechnungsformel gemäß dem Verformungsgrad und der Verformungsdauer des Erfassungspunkts und eines Werts von Arbeit an dem Erfassungspunkt während einer tatsächlichen Erfassung unter jedem Übungstyp, um eine Beziehung zwischen dem Übungstyp, dem Erfassungspunkt und einer Berechnungsformel, die bestimmte Koeffizienten aufweist, zu erhalten,
wobei der Schritt des Bestimmens des Werts von Arbeit durch den Benutzer auf der elastischen Matte an jedem Erfassungspunkt der elastischen Matte gemäß dem Verformungsgrad und der Verformungsdauer von jedem voreingestellten Erfassungspunkt der Oberfläche der elastischen Matte und der Berechnungsformel, die bestimmte Koeffizienten aufweist und jedem Erfassungspunkt entspricht, umfasst:
Beziehen eines aktuellen Übungstyps,
Bestimmen der Berechnungsformel, die bestimmte Koeffizienten aufweist und jedem Erfassungspunkt entspricht, gemäß dem Übungstyp, den Erfassungspunkten und der Berechnungsformel, die bestimmte Koeffizienten aufweist, unter dem aktuellen Übungstyp, und
Bestimmen des Werts von Arbeit durch den Benutzer auf der elastischen Matte an jedem Erfassungspunkt der elastischen Matte gemäß dem Verformungsgrad und der Verformungsdauer von jedem voreingestellten Erfassungspunkt der Oberfläche der elastischen Matte und der Berechnungsformel, die bestimmte Koeffizienten aufweist und jedem Erfassungspunkt entspricht, unter dem aktuellen Übungstyp.

4. Verfahren nach Anspruch 3, wobei der Schritt des Beziehens des Übungstyps umfasst:
Bestimmen des aktuellen Übungstyps gemäß den Verformungsinformationen der Oberfläche der elastischen Matte oder
Beziehen des aktuellen Übungstyps, der von dem Benutzer eingestellt wurde.

5. Verfahren nach Anspruch 1, ferner umfassend:
Akkumulieren von Werten von Energie, die für viele Male bezogen wurden und, wenn ein akkumulierter Wert eine voreingestellte Schwelle erreicht, Absenden eines Aufforderu ngssig nals.

6. Verfahren nach Anspruch 1, wobei nach dem Aufzeichnen des Werts von Energie das Verfahren ferner umfasst:
Übertragen des Werts von Energie an ein eingebundenes Zielendgerät, um dem Zielendgerät zu ermöglichen, Statistiken zu erstellen und den Wert von Energie anzuzeigen.

7. Vorrichtung zur Bestimmung eines Werts verbrauchter Energie, umfassend:
ein Bezugsmodul (710), das dazu ausgestaltet ist, Verformungsinformationen einer Oberfläche einer elastischen Matte zu beziehen,
ein Bestimmungsmodul (720), das dazu ausgestaltet ist, einen Wert der Energie, die von einem Benutzer verbraucht wird, wenn eine entsprechende Verformung erzeugt wird, gemäß den Verformungsinformationen der Oberfläche der elastischen Matte zu bestimmen, und
ein Aufzeichnungsmodul (730), das dazu ausgestaltet ist, den Wert der Energie aufzuzeichnen, wobei die Verformungsinformationen einen Verformungsgrad und eine Verformungsdauer von jedem voreingestellten Erfassungspunkt der Oberfläche der elastischen Matte umfassen,
**dadurch gekennzeichnet, dass** der Verformungsgrad die Entfernung von einer Anfangsposition zu einer maximalen Verformungsposition der Oberfläche der elastischen Matte an dem Erfassungspunkt während eines Verformungsvorgangs ist, und die Verformungsdauer eine Dauer ist, in der die Oberfläche der elastischen Matte an dem Erfassungspunkt aus der Anfangsposition zu der maximalen Verformungsposition während eines Verformungsvorgangs verformt wird,
und wobei das Bestimmungsmodul (720) ausgestaltet ist zum:
Bestimmen eines Werts von Arbeit durch den Benutzer auf der elastischen Matte an jedem Erfassungspunkt der elastischen Matte gemäß dem Verformungsgrad und der Verformungsdauer von jedem voreingestellten Erfassungspunkt der Oberfläche der elastischen Matte, und
Bestimmen der Summe von Werten von Arbeit, die allen voreingestellten Erfassungspunkten entsprechen, als der Wert der Energie, die von dem Benutzer verbraucht wird.

8. Vorrichtung nach Anspruch 7, ferner umfassend:
ein Aufforderungsmodul (760), das dazu ausgestaltet ist, Werte von Energie, die für viele Male bezogen wurden, zu akkumulieren, und wenn ein akkumulierter Wert eine voreingestellte Schwelle erreicht, ein Aufforderungssignal abzusenden, und/oder
ein Übertragungsmodul (770), das dazu ausgestaltet ist, den Wert von Energie an ein eingebundenes Zielendgerät zu übertragen, um dem Zielendgerät zu ermöglichen, Statistiken zu erstellen und den Wert von Energie anzuzeigen.

9. Computerprogramm, das Anweisungen zum Ausführen der Schritte eines Verfahrens zur Bestimmung eines Werts von verbrauchter Energie nach einem der Ansprüche 1 bis 6, wenn das Programm von einem Computer ausgeführt wird, beinhaltet.

10. Aufzeichnungsmedium, das von einem Computer lesbar ist und auf dem ein Computerprogramm aufgezeichnet ist, das Anweisungen zum Ausführen der Schritte eines Verfahrens zur Bestimmung eines Werts von verbrauchter Energie nach einem der Ansprüche 1 bis 6 beinhaltet.

## Revendications

1. Procédé de détermination d'une valeur d'énergie consommée, comprenant les étapes consistant à :
acquérir (101), par un composant de traitement d'un tapis élastique, des informations de déformation d'une surface du tapis élastique ;
déterminer (102), par le composant de traitement du tapis élastique, selon les informations de déformation de la surface du tapis élastique, une valeur d'énergie consommée par un utilisateur lorsqu'une déformation correspondante est générée ; et
enregistrer (103), par le composant de traitement du tapis élastique, la valeur d'énergie,
**caractérisé en ce que** les informations de déformation comprennent un degré de déformation et une durée de déformation de chaque point de détection prédéfini de la surface du tapis élastique,
dans lequel le degré de déformation est la distance entre une position initiale et une position de déformation maximale de la surface du tapis élastique au point de détection au cours d'un processus de déformation ; et la durée de déformation est une durée pendant laquelle la surface du tapis élastique au point de détection se déforme de la position initiale à la position de déformation maximale au cours d'un processus de déformation, et
dans lequel l'étape consistant à déterminer (102), par le composant de traitement du tapis élastique, selon les informations de déformation de la surface du tapis élastique, la valeur de l'énergie consommée par l'utilisateur lorsque la déformation correspondante est générée comprend :
la détermination, selon le degré de déformation et la durée de déformation de chaque point de détection prédéfini de la surface du tapis élastique, d'une valeur de travail de l'utilisateur sur le tapis élastique à chaque point de détection du tapis élastique ; et
la détermination de la somme de valeurs de travail correspondant à tous les points de détection prédéfinis comme étant la valeur de l'énergie consommée par l'utilisateur.

2. Procédé selon la revendication 1, dans lequel le procédé comprend en outre les étapes consistant à :
en se basant sur des degrés de déformations, des durées de déformations et des coefficients d'ajustement prédéfinis, élaborer (201) une formule de calcul du travail sur des points de détection du tapis élastique ;
déterminer (202), pour chaque point de détection du tapis élastique, des valeurs des coefficients d'ajustement de la formule de calcul, selon le degré de déformation et la durée de déformation du point de détection, et une valeur de travail sur le point de détection lors d'une détection réelle, de manière à obtenir une formule de calcul ayant des coefficients déterminés et correspondant à chaque point de détection ;
l'étape consistant à déterminer, selon le degré de déformation et la durée de déformation de chaque point de détection prédéfini de la surface du tapis élastique, la valeur de travail de l'utilisateur sur le tapis élastique à chaque point de détection du tapis élastique comprend :
la détermination (203), selon le degré de déformation et la durée de déformation de chaque point de détection prédéfini de la surface du tapis élastique, et selon la formule de calcul ayant des coefficients déterminés et correspondant à chaque point de détection, de la valeur de travail de l'utilisateur sur le tapis élastique à chaque point de détection du tapis élastique.

3. Procédé selon la revendication 2, dans lequel l'étape consistant à déterminer, pour chaque point de détection du tapis élastique, des valeurs des coefficients d'ajustement de la formule de calcul, selon le degré de déformation et la durée de déformation du point de détection, et la valeur de travail sur le point de détection lors d'une détection réelle, de manière à obtenir la formule de calcul ayant des coefficients déterminés et correspondant à chaque point de détection comprend :
la détermination, pour chaque point de détection du tapis élastique, de valeurs des coefficients d'ajustement de la formule de calcul, selon le degré de déformation et la durée de déformation du point de détection, et d'une valeur de travail sur le point de détection lors d'une détection réelle dans le cadre de chaque type d'exercice, de manière à obtenir une relation entre le type d'exercice, le point de détection et une formule de calcul ayant des coefficients déterminés ;
l'étape consistant à déterminer, selon le degré de déformation et la durée de déformation de chaque point de détection prédéfini de la surface du tapis élastique, et selon la formule de calcul ayant des coefficients déterminés et correspondant à chaque point de détection, la valeur de travail de l'utilisateur sur le tapis élastique à chaque point de détection du tapis élastique comprend :
l'acquisition d'un type d'exercice en cours ;
selon la relation entre le type d'exercice, les points de détection et la formule de calcul ayant des coefficients déterminés, la détermination de la formule de calcul ayant des coefficients déterminés et correspondant à chaque point de détection dans le cadre du type d'exercice en cours ; et
la détermination, selon le degré de déformation et la durée de déformation de chaque point de détection prédéfini de la surface du tapis élastique, et selon la formule de calcul ayant des coefficients déterminés et correspondant à chaque point de détection dans le cadre du type d'exercice en cours, de la valeur de travail de l'utilisateur sur le tapis élastique à chaque point de détection du tapis élastique.

4. Procédé selon la revendication 3, dans lequel l'étape consistant à acquérir le type d'exercice comprend :
la détermination, selon les informations de déformation de la surface du tapis élastique, du type d'exercice en cours ; ou,
l'acquisition du type d'exercice en cours défini par l'utilisateur.

5. Procédé selon la revendication 1, comprenant en outre les étapes consistant à :
accumuler des valeurs d'énergie acquises plusieurs fois, et lorsqu'une valeur accumulée atteint un seuil prédéfini, envoyer un signal d'invite.

6. Procédé selon la revendication 1, dans lequel, après l'enregistrement de la valeur d'énergie, le procédé comprend en outre les étapes consistant à :
transmettre la valeur d'énergie à un terminal cible lié de manière à permettre au terminal cible d'établir des statistiques et d'afficher la valeur d'énergie.

7. Dispositif pour déterminer une valeur d'énergie consommée, comprenant :
un module d'acquisition (710), configuré pour acquérir des informations de déformation d'une surface d'un tapis élastique ;
un module de détermination (720), configuré pour déterminer, selon les informations de déformation de la surface du tapis élastique, une valeur d'énergie consommée par un utilisateur lorsqu'une déformation correspondante est générée ; et
un module d'enregistrement (730), configuré pour enregistrer la valeur d'énergie, dans lequel les informations de déformation comprennent un degré de déformation et une durée de déformation de chaque point de détection prédéfini de la surface du tapis élastique,
**caractérisé en ce que** le degré de déformation est la distance entre une position initiale et une position de déformation maximale de la surface du tapis élastique au point de détection au cours d'un processus de déformation ; et la durée de déformation est une durée pendant laquelle la surface du tapis élastique au point de détection se déforme de la position initiale à la position de déformation maximale au cours d'un processus de déformation,
et dans lequel le module de détermination (720) est configuré pour :
déterminer, selon le degré de déformation et la durée de déformation de chaque point de détection prédéfini de la surface du tapis élastique, une valeur de travail de l'utilisateur sur le tapis élastique à chaque point de détection du tapis élastique ; et
déterminer la somme de valeurs de travail correspondant à tous les points de détection prédéfinis comme étant la valeur de l'énergie consommée par l'utilisateur.

8. Dispositif selon la revendication 7, comprenant en outre :
un module d'invite (760), configuré pour accumuler des valeurs d'énergie acquises plusieurs fois, et lorsqu'une valeur accumulée atteint un seuil prédéfini, envoyer un signal d'invite ; et/ou
un module de transmission (770), configuré pour transmettre la valeur d'énergie à un terminal cible lié, de manière à permettre au terminal cible d'établir des statistiques et d'afficher la valeur d'énergie.

9. Programme informatique comportant des instructions pour exécuter les étapes d'un procédé de détermination d'une valeur d'énergie consommée selon l'une quelconque des revendications 1 à 6, lorsque ledit programme est exécuté par un ordinateur.

10. Support d'enregistrement lisible par un ordinateur et sur lequel est enregistré un programme informatique comportant des instructions pour exécuter les étapes d'un procédé de détermination d'une valeur d'énergie consommée selon l'une quelconque des revendications 1 à 6.
